# EUROPEAN PATENT SPECIFICATION

(11) **EP 2 739 621 B1**
(45) Date of publication and mention of the grant of the patent: **21.06.2017**
(21) Application number: 12759833.2
(22) Date of filing: 01.08.2012
(51) Int. Cl.: C07D 417/14, A61K 31/496, A61P 7/04

(54) **2-ACYLAMINOTHIAZOLE COMPOUND CRYSTALS**
KRISTALLE MIT EINER 2-ACYLAMINOTHIAZOL-VERBINDUNG
CRISTAUX DE COMPOSÉ DE 2-ACYLAMINOTHIAZOLE

(30) Priority: 03.08.2011 JP 2011169730
(43) Date of publication of application: 11.06.2014
(73) Proprietor: Astellas Pharma Inc., Tokyo 103-8411 (JP)
(72) Inventor: SUGASAWA, Keizo, Tokyo 103-8411 (JP); MIYAFUJI, Akio, Tokyo 103-8411 (JP); SUZUKI, Kenichi, Tokyo 103-8411 (JP); AWAMURA, Yuji, Tokyo 103-8411 (JP)
(74) Representative: Hoffmann Eitle
(86) International application number: PCT/JP2012/004885
(87) International publication number: WO 2013/018362

(56) References cited:
- WO-A1-2004/029049

## Description

### Technical Field

The present invention relates to 2-acylaminothiazole compound crystals that are useful as an active ingredient in pharmaceutical compositions, such as pharmaceutical compositions for treating thrombocytopenia.

### Background Art

1-(3-chloro-5-{[4-(4-chlorothiophen-2-yl)-5-(4-cyclohexylpiperazin-1-yl)thiazol-2-yl]carbamoyl}pyridin-2-yl)piperidine-4-carboxylic acid as expressed by the following chemical formula (hereinafter referred to as "Compound X") and pharmaceutically acceptable salts are known to have excellent thrombocytosis effects (patent literature 1, patent literature 2).

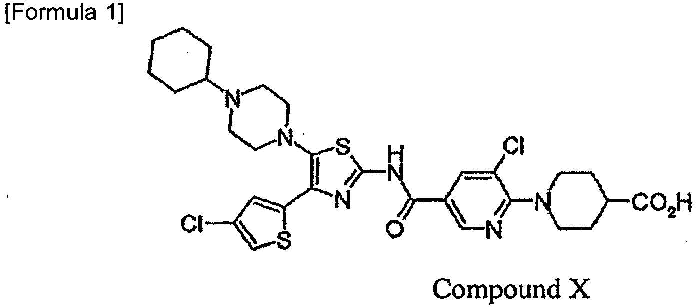

Patent literature 1 discloses a hydrochloride of compound X as example 16 (hereinafter referred to as "compound X hydrochloride").

Furthermore, patent literature 2 discloses a maleic acid salt of compound X that has endothermic peaks near 198 degree C and 271 degree C in thermo gravimetric analysis (hereinafter referred to as "maleic acid salt of compound X"). However, patent literature 2 neither discloses nor suggests that the maleic acid salt of compound X exhibits crystal polymorphism.

On the other hand, compounds exhibiting crystal polymorphism demonstrate entirely different effects regardless of being the same compound, because various physical properties including physicochemical properties differ depending on the crystalline form. In pharmaceutical products in particular, if compounds that have different functional effects are expected to have the same effect, a different functional effect than expected will occur, which is thought to induce unexpected circumstances, and therefore there is demand for supply of a drug substance with constant quality. Therefore, when a compound which has crystal polymorphism is used as a medicine, one type of crystal of that compound must always be constantly provided in order to ensure constant quality and constant effects that are required of the medicine.

Under the aforementioned conditions, from the perspective of supplying a drug substance for medicines, there is a need for compound X or crystals of pharmaceutically acceptable salts thereof, which can ensure constant quality and constant effects and which can be stably supplied in mass production such as industrial production or the like, as well as for establishment of a manufacturing method thereof.

### Citation List

### Patent Literature

PTL 1: International patent publication WO 03/062233
PTL 2: International patent publication WO 2004/029049

### Summary of Invention

### Technical Problem

The crystals of compound X maleic acid salt disclosed in patent literature 2 (hereinafter referred to as "compound X maleic acid salt A type crystals") cannot be isolated as compound X maleic acid salt A type crystals when scaled up for mass production using the method disclosed in example 1 of patent literature 2, and therefore must be isolated in a different crystal form. (This other crystal form is referred to as "compound X maleic acid salt B type crystals"). Therefore, the compound X maleic acid salt A type crystals have a possibility that the crystal form will morph depending on the scale of production, and is clearly inappropriate as a drug substance for medicines which require constant quality and constant effects.

Therefore, the present invention provides a crystal of compound X maleic acid salt that can ensure constant quality and constant effects as a drug substance for medicines, and that can be stably supplied in mass production such as industrial production.

### Solution to Problem

As a result of diligent research concerning compound X maleic acid salt crystals, the present inventors discovered that compound X maleic acid salt crystals exhibit crystal polymorphism, and of these forms, C type crystals (hereinafter referred to as "compound X maleic acid salt C type crystals") have excellent oral absorption, ensure constant quality even in mass production such as industrial production, and can be stably supplied, and thus the present invention was achieved.

In other words, the present invention relates to compound X maleic acid salt C type crystals, and to a pharmaceutical composition containing compound X maleic acid salt C type crystals and excipients.

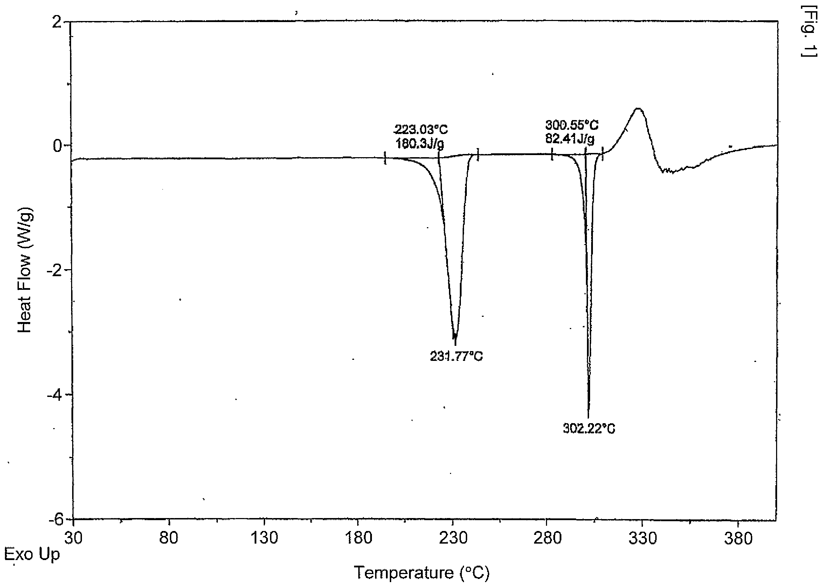

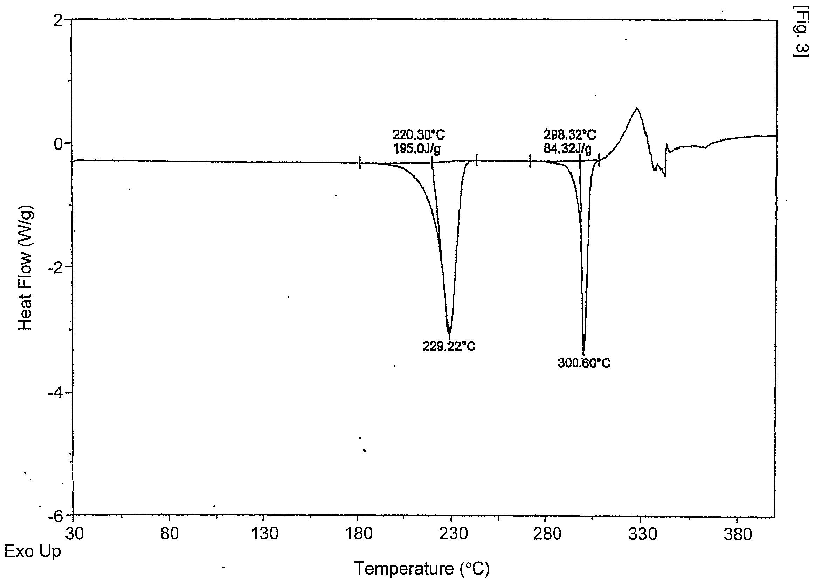

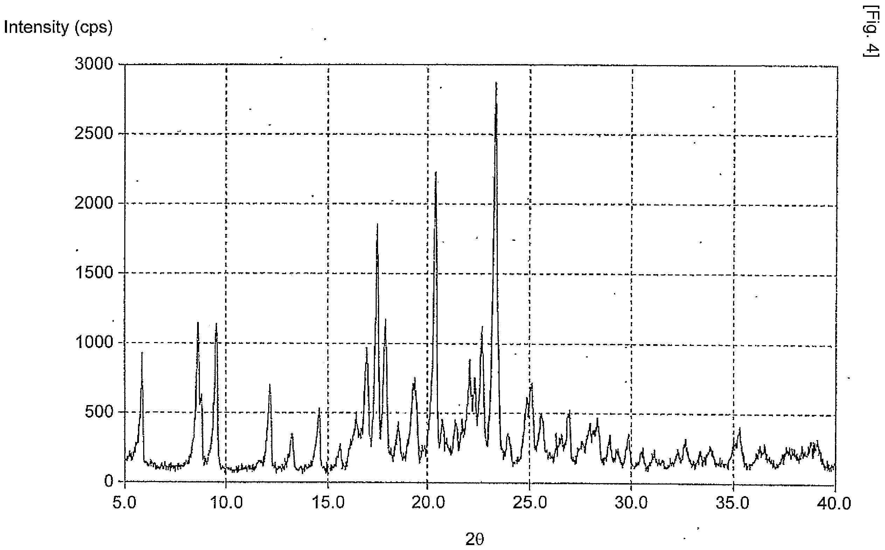

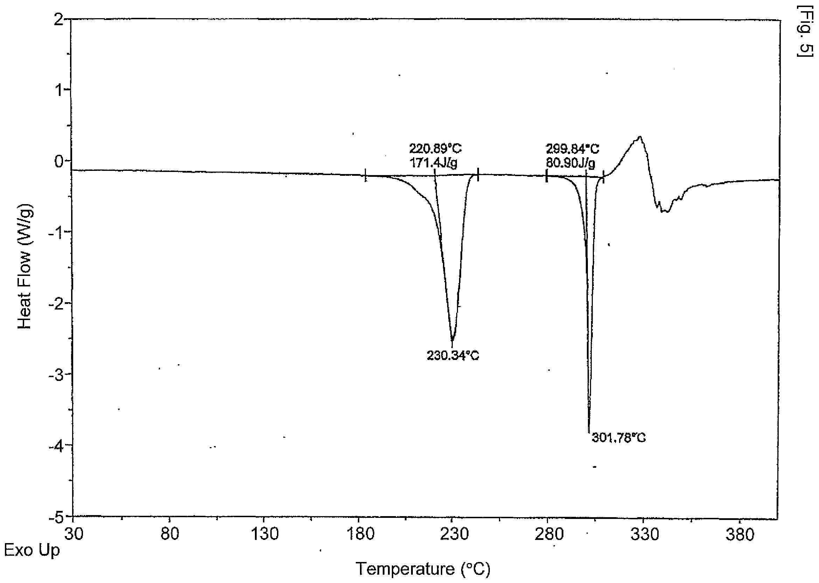

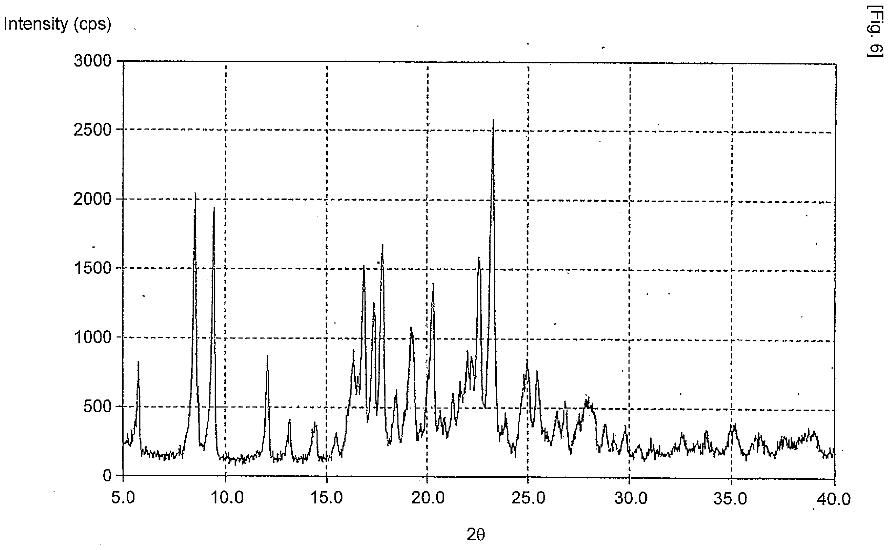

As illustrated in FIG. 1 through FIG. 6, compound X maleic acid salt C type crystals are characterized by the following parameters.

### Differential Scanning Calorimeter Analysis

The endothermic peak top temperatures are between 229 and 232 degree C and between 300 and 303 degree C.

Other embodiments include the differential scanning calorimeter analysis charts shown in FIG. 1, FIG. 3, FIG. 5, as well as other charts that are recognized by one skilled in the art as being essentially identical to them.

### Powder X-Ray Crystal Diffraction

When measured using a Cu-K alpha beam, peaks are observed at 8.5 degree, 9.5 degree, 12.1 degree, 20.4 degree, 22.7 degree, and 23.3 degree, for a diffraction angle of 2-theta.

As another embodiment, when measured using a Cu-K alpha beam, peaks are observed at 5.8 degree, 8.5 degree, 9.5 degree, 12.1 degree, 16.9 degree, 17.4 degree, 17.8 degree, 19.3 degree, 20.4 degree, 22.7 degree, and 23.3 degree for a diffraction angle of 2-theta.

As yet another embodiment, when measured using a Cu-K alpha beam, peaks are observed at 8.4 degree - 8.8 degree, 9.3 degree - 9.7 degree, 11.9 degree - 12.3 degree, 20.2 degree - 20.6 degree, 22.5 degree - 22.9 degree, 23.1 degree - 23.5 degree, for a diffraction angle of 2-theta.

As yet another embodiment, when measured using a Cu-K alpha beam, peaks are observed at 5.6 degree - 6.0 degree, 8.4 degree - 8.8 degree, 9.3 degree - 9.7 degree, 11.9 degree - 12.3 degree, 16.7 degree - 17.1 degree, 17.2 degree - 17.6 degree, 17.6 degree - 18.0 degree, 19.1 degree - 19.5 degree, 20.2 degree - 20.6 degree, 22.5 degree - 22.9 degree, 23.1 degree - 23.5 degree, for a diffraction angle of 2-theta.

As yet another embodiment, when measured using a Cu-K alpha beam, peaks are observed at 8.5 degree - 8.7 degree, 9.4 degree - 9.6 degree, 12.0 degree- 12.2 degree, 20.3 degree - 20.5 degree, 22.5 degree - 22.8 degree, 23.2 degree - 23.4 degree, for a diffraction angle of 2-theta.

As yet another embodiment, when measured using a Cu-K alpha beam, peaks are observed at 5.7 degree - 5.9 degree, 8.5 degree - 8.7 degree, 9.4 degree - 9.6 degree, 12.0 degree- 12.2 degree, 16.8 degree - 17.0 degree, 17.3 degree - 17.5 degree, 17.7 degree - 17.9 degree, 19.2 degree - 19.4 degree, 20.3 degree - 20.5 degree, 22.5 degree - 22.8 degree, 23.2 degree - 23.4 degree, for a diffraction angle of 2-theta.

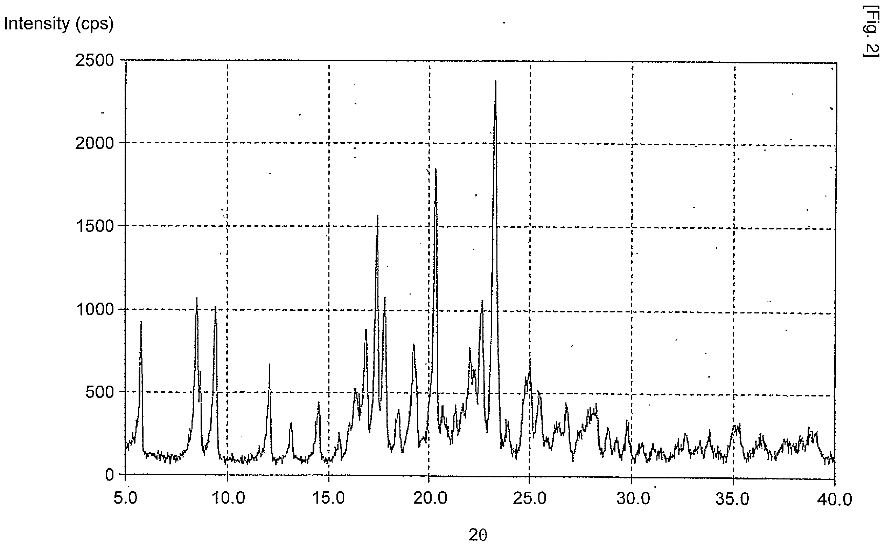

Other embodiments include the powder x-ray crystal diffraction patterns shown in FIG. 2, FIG. 4, FIG. 6, as well as other patterns that are recognized by one skilled in the art as being essentially identical to them.

Note, with powder x-ray crystal diffraction, the crystal diffraction angle and the overall pattern are important data characteristics when verifying the identity of crystals, and the relative strength can change somewhat depending on the direction of crystal growth, particle size, and measurement conditions, and therefore should not be interpreted strictly. Furthermore, the present invention relates to pure compound X maleic acid salt C type crystals, but mixtures that can appear essentially identical to pure compound X maleic acid salt C type crystals are also included in the present invention.

Therefore, the present invention relates to compound X maleic acid salt crystals that are characterized in differential scanning calorimeter analysis by having endothermic peak top temperatures between 229 and 232 degree C and between 300 and 303 degree C. As another embodiment, the present invention relates to compound X maleic acid salt crystals that are characterized in powder x-ray crystal diffraction using a Cu-K alpha beam by having peaks at 8.5 degree, 9.5 degree, 12.1 degree, 20.4 degree, 22.7 degree, and 23.3 degree, for a diffraction angle of 2-theta. As yet another embodiment, the present invention relates to compound X maleic acid salt crystals that are characterized in powder x-ray crystal diffraction using a Cu-K alpha beam by having peaks at 5.8 degree, 8.5 degree, 9.5 degree, 12.1 degree, 16.9 degree, 17.4 degree, 17.8 degree, 19.3 degree, 20.4 degree, 22.7 degree, and 23.3 degree, for a diffraction angle of 2-theta. As another embodiment, the present invention relates to compound X maleic acid salt crystals that are characterized in differential scanning calorimeter analysis by having endothermic peak top temperatures between 229 and 232 degree C and between 300 and 303 degree C, and in powder x-ray crystal diffraction using a Cu-K alpha beam by having peaks at 8.5 degree, 9.5 degree, 12.1 degree, 20.4 degree, 22.7 degree, and 23.3 degree, for a diffraction angle of 2-theta. As yet another embodiment, the present invention relates to compound X maleic acid salt crystals that are characterized in differential scanning calorimeter analysis by having endothermic peak top temperatures between 229 and 232 degree C and between 300 and 303 degree C, and in powder x-ray crystal diffraction using a Cu-K alpha beam by having peaks at 5.8 degree, 8.5 degree, 9.5 degree, 12.1 degree, 16.9 degree, 17.4 degree, 17.8 degree, 19.3 degree, 20.4 degree, 22.7 degree, and 23.3 degree, for a diffraction angle of 2-theta.

Furthermore, the present invention relates to a pharmaceutical composition for treating thrombocytopenia that comprises compound X maleic acid salt C type crystals. Note, this pharmaceutical composition includes thrombocytopenia treatment agents that comprise compound X maleic acid salt C type crystals. Furthermore, this pharmaceutical composition can include any pharmaceutical compositions comprising compound X maleic acid salt C type crystals while maintaining the crystal form, and for example, a solid drug composition can be exemplified. In addition, this pharmaceutical composition also includes liquid pharmaceutical compositions containing the compound X maleic acid salt C type crystals while maintaining the crystal form, such as a suspension agent.

Furthermore, the present invention relates to the use of compound X maleic acid salt C type crystals for manufacturing a pharmaceutical composition for treating thrombocytopenia, the use of compound X maleic acid salt C type crystals for treating thrombocytopenia, compound X maleic acid salt C type crystals for treating thrombocytopenia, and a method of treating thrombocytopenia by administering to a subject an effective dose of compound X maleic acid salt C type crystals. Note, a "subject" refers to a human or other animal that requires treatment, and one embodiment is a human that requires treatment.

### Advantageous Effects of Invention

Compound X maleic acid salt C type crystals exhibit excellent oral absorption, ensure constant quality even during mass production such as industrial production, and can be stably supplied. Furthermore, compound X maleic acid salt C type crystals can be used as an active ingredient for a pharmaceutical composition for treating thrombocytopenia.

### Brief Description of Drawings

[Fig. 1] FIG. 1 is a differential scanning calorimeter analysis chart of compound X maleic acid, salt C type crystals manufactured in accordance with the method of example 1.
[Fig. 2] FIG. 2 is a powder X-ray crystal diffraction pattern of compound X maleic acid salt C type crystals manufactured in accordance with the method of example 1.
[Fig. 3] FIG. 3 is a differential scanning calorimeter analysis chart of compound X maleic acid C type crystals manufactured in accordance with the method of example 2.
[Fig. 4] FIG. 4 is a powder X-ray crystal diffraction pattern of compound X maleic acid salt C type crystals manufactured in accordance with the method of example 2.
[Fig. 5] FIG. 5 is a differential scanning calorimeter analysis chart of compound X maleic acid C type crystals manufactured in a manner similar to the method of example 3.
[Fig. 6] FIG. 6 is a powder X-ray crystal diffraction pattern of compound X maleic acid salt C type crystals manufactured in a manner similar to the method of example 3.
[Fig. 7] FIG. 7 is a differential scanning calorimeter analysis chart of compound X maleic acid A type crystals manufactured in accordance with the method disclosed in patent literature 2.

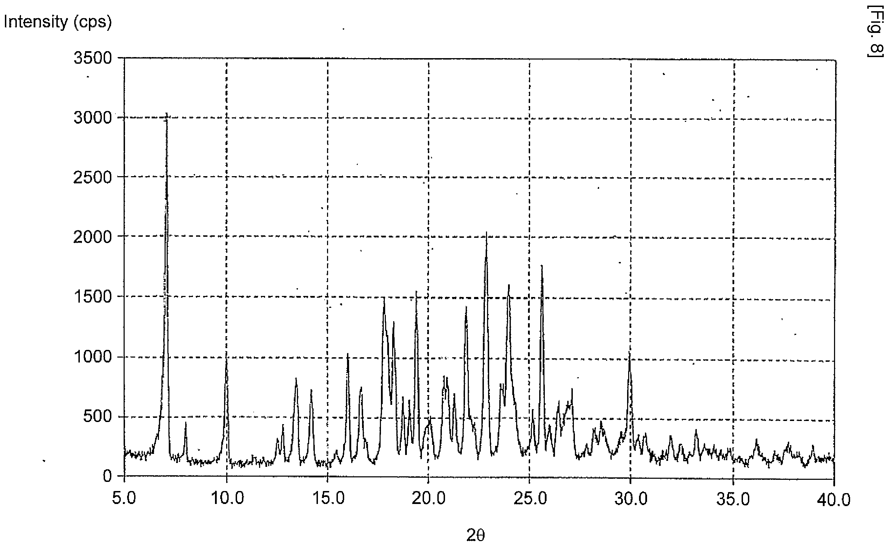

[Fig. 8] FIG. 8 is a powder X-ray crystal diffraction pattern of compound X maleic acid salt A type crystals manufactured in accordance with the method disclosed in patent literature 2.
[Fig. 9] FIG. 9 is a differential scanning calorimeter analysis chart of compound X maleic acid B type crystals manufactured in accordance with the method of preparation example 1.

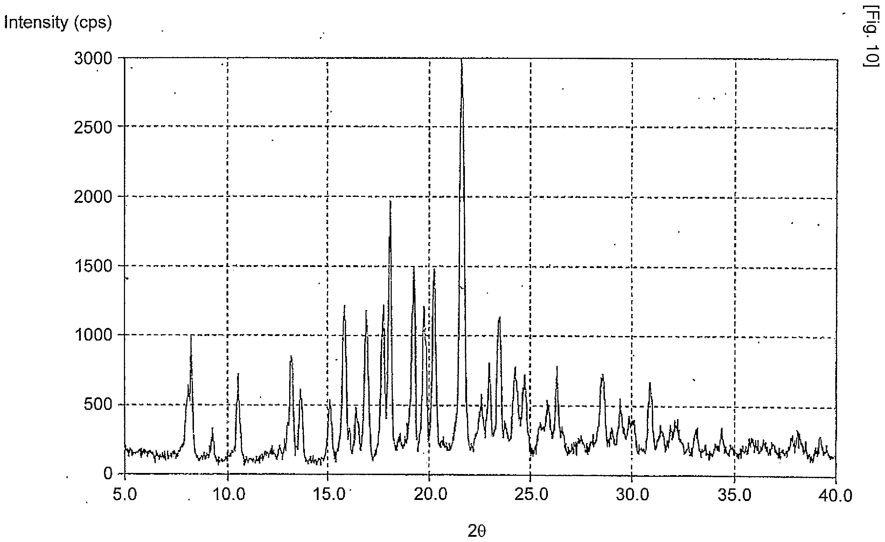

[Fig. 10] FIG. 10 is a powder X-ray crystal diffraction pattern of compound X maleic acid salt B type crystals manufactured in accordance with the method of preparation example 1.

### Description of Embodiments

The present invention will be described below in detail.

The values obtained from the various spectrums may have some error due to the crystal growth direction, particle size, and measurement conditions. Therefore, values of the endothermic peak top temperature in differential scanning calorimeter analysis mean that a range of ± 3 degree C from that value is acceptable, in another embodiment, means that a range of ± 2 degree C from that value is acceptable, and in yet another embodiment, means that a range of ± 1 degree C from that value is acceptable. Furthermore, the term "" that is used for the value of the diffraction angle 2-theta in powder X-ray crystal diffraction means that the value is for a diffraction angle of 2-theta, and in one embodiment, means that a range of ± 1 degree is acceptable, in another embodiment, means that a range of ± 0.5 degree is acceptable, and in yet another embodiment, means that a range of ± 0.2 degree is acceptable.

When manufacturing the compound X maleic acid salt crystals, the necessary starting compounds can be obtained as described below. In other words, compound X or a pharmaceutically acceptable salt thereof can be obtained by the method according to patent literature 1, a variation method thereof, or an obvious method to one skilled in the art, and the compound X maleic acid salt can be obtained by the method according to patent literature 2, a variation method thereof, or an obvious method to one skilled in the art.

Compound X maleic acid salt A type crystals and compound X maleic acid salt B type crystals can be obtained by crystallizing in a mixture of water and alcohol such as methanol, ethanol, isopropanol, and in another embodiment, can be obtained by crystallizing in 80% ethanol water, but on the other hand, compound X maleic acid salt C type crystal can be obtained by the following method.

In other words, compound X maleic acid salt C type crystal can be obtained by crystallizing in: a mixture of water and acetone, methyl ethyl ketone, or acetonitrile, which may also contain dimethylsulfoxide (DMSO): or in another embodiment, in 50% or higher acetone water which may contain DMSO; or in yet another embodiment, in 60% or higher acetone water which may contain DMSO; or in yet another embodiment, in a mixture where acetone: water: DMSO = 1 - 3:0.1 - 2:1 - 3, or in another embodiment, in a mixture where acetone: water: DMSO = 2:1:2.

The pharmacological activity of the compound X maleic acid salt C type crystals was confirmed by the following tests.

### Test Example 1 Cell Proliferation Test for Human c-mpl-Ba/F3 Cells

Human c-mpl-Ba/F3 cells at a concentration of 2 x 10⁵ cells/mL were cultured at 37 degree C on a 96 well microplate using RPMI 1640 culture medium (100 microliter/well) containing 10% fetal calf serum to which various concentrations of the test compound was added. After 24 hours from the start of culturing, 10 microliter/well of WST-1/1-methoxy PMS (cell measuring kit, Dojin) was added. Immediately after the addition and two hours later, the A450/A650 absorbance was measured using a microplate reader (Model 3350, Bio-Rad), and the increase in absorbance after 2 hours was interpreted to represent the proliferation activity of each test compound.

The results showed that the cell proliferation effect of compound X maleic acid salt C type crystal on human c-mpl-Ba/F3 cells was 2.0 nM at EC₅₀ Furthermore, the maximum cell proliferation activity of compound X maleic acid salt C type crystals was 102%, based on the maximum cell proliferation activity of rhTPO being 100%.

Furthermore, when Ba/F3 cells where c-mpl was not introduced were used, a cell proliferation effect was not observed, and therefore it was confirmed that compound X maleic acid salt C type crystals affect the proliferation of Ba/F3 cells through human c-mpl.

The results of the aforementioned test clearly showed that, compound X maleic acid salt C type crystals have a thrombocytosis effect. Therefore, compound X maleic acid salt C type crystals are useful for treating and preventing various types of thrombocytopenia by having a thrombocytosis effect. Herein, the various types of thrombocytopenia include anaplastic anemia, thrombocytopenia during myelodysplastic syndrome, thrombocytopenia due to chemotherapy or radiation therapy of malignant tumors, idiopathic thrombocytopenic purpura, thrombocytopenia during hepatic disease, thrombocytopenia due to HIV. Furthermore, compound X maleic acid salt C type crystals have thrombocytosis effects, and therefore can prevent thrombocytopenia by being administered before performing various treatments including chemotherapy or radiation therapy where there is a possibility of causing thrombocytopenia.

A pharmaceutical composition containing compound X maleic acid salt C type crystals as an active ingredient can be prepared by a commonly used method using excipients that are normally used in this field, or in other words using excipients for drugs and carriers for drugs.

Administration can be either by oral administration using tablets, pills, capsules, granules, powders, or liquid, or by a non-oral administration using intra-articular, intravenous, or intramuscular injections, suppositories, eyedrops, eye ointments, transdermal liquids, ointments, transdermal patches, transmucosal liquids, transmucosal patches, and inhalants.

A solid composition for oral administration can be used in tablet, powder, and granule drugs. In these solid compositions, one or more type of active ingredient is blended with at least one type of inactive excipient. The composition may contain inert additives such as lubricating agents, disintegrating agents, stabilizers, and solubilizing agents, in accordance with standard methods. Tablets and pills can be coated with a sugar coating or with a gastric dissolving or enteric film, if necessary.

Liquid compositions for oral administration include pharmacologically acceptable emulsions, solutions, suspensions, syrups or elixirs, and contain commonly used inert diluents such as purified water or ethanol. These liquid compositions can also contain sweeteners, flavorings, fragrances, preservatives, and adjuvants such as wetting agents and suspending agents, in addition to the inert diluent.

Injections for non-oral administration contain sterile aqueous or non-aqueous solutions, suspending agents, or emulsifiers. Examples of aqueous solutions include distilled water for injection and physiological saline solutions. Examples of non-aqueous solvents include alcohols such as ethanol. These compositions can also contain preservatives, wetting agents, emulsifiers, dispersing agents, stabilizers, or solubilizing agents. These agents are sterilized for example by filtering through a bacteria retaining filter, blending with a sterilizing agent, or sterilizing by irradiation. Furthermore, these compositions can be used by preparing a sterile solid composition, and dissolving or suspending in sterile water or a sterile solvent for injection prior to use.

For the case of normal oral administration, a daily dose is between 0.001 and 50 mg/kg of body weight, preferably between 0.1 and 10 mg/kg, and this dose is administered at one time or is divided between 2 to 4 times. For the case of intravenous administration, a daily dose of 0.0001 to 1 mg/kg is suitable, and this dose is administered one time per day, or is divided over several administrations. The dose can be appropriately determined based on individual cases, considering the symptoms, age, and sex.

The pharmaceutical composition of the present invention contains 0.1 to 100 weight %, or in another aspect, 0.01 to 50 weight %, of compound X maleic acid salt C type crystal which is the active ingredient, but this can vary depending on administration route, drug form, administration site, and type of excipients or additives.

The compound X maleic acid salt C type crystals can be used in combination with other types of treatments or preventative agents for the aforementioned conditions that compound X maleic acid salt C type crystals are thought to be effective towards. This combined use can be administered simultaneously, or administered separately but continuously, or administered with a predetermined time interval. Simultaneously administered drugs can be manufactured as a mixed or fixed dose, or can be manufactured separately.

### Example

The compound X maleic acid salt C type crystal manufacturing method is described below in further detail based on examples. Note, the manufacturing method of the compound X maleic acid salt C type crystals is not restricted to only the manufacturing method specifically shown in the following examples, and manufacturing can also be performed by alternate methods of the manufacturing method shown below, or by methods that are obvious to one skilled in the art.

Note, for convenience, concentration mol/L is expressed as M. For example, a 1 M sodium hydroxide aqueous solution refers to a 1 mol/L aqueous solution of sodium hydroxide.

The differential scanning calorimeter analysis was conducted on a test sample of 5 mg using a TA Instruments TA 5000 (2910 AutoDSC) between room temperature and 300 degree C or between room temperature and 400 degree C (temperature increase rate: 10 degree C/minute), under N₂ (flow rate: 50 mL/minute) using an aluminum sample pan.

Furthermore, powder x-ray crystal diffraction was measured using a MXP18TAHF22 under conditions of tube: Cu, tube current: 40 mA, tube voltage: 40 kV, sampling width: 0.020 degree, scanning rate: 3 degree/minute, wavelength: 1.54056 angstrom, measurement diffraction angle range (2-theta): 3 to 40 degree or 5 to 40 degree.

### Preparation Example 1: Manufacture of Compound X Maleic Acid Salt B Type Crystal

310 mL of a 1 M aqueous solution of sodium hydroxide at room temperature was added to a mixture of 70.0 g of the ethyl ester of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid and 1.2 L of ethanol, the insoluble matter was filtered out, and then washed with 200 mL of ethanol. The reaction solution was stirred for 90 minutes at 60 degree C. After cooling to room temperature, 1.4 L of an aqueous solution containing 24.11 g of maleic acid was added to the solution obtained, and then the precipitate was collected by filtering.

The same operation was repeated and when combined with the previously obtained precipitate, 136.05 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid was obtained.

18.9 g of maleic acid and 2.1 L of 80% ethanol water were added to 88.90 g of the carboxylic acid obtained, and the solution was stirred for one hour at room temperature and for another hour at 100 degree C. After cooling to room temperature and further cooling with ice, the precipitated solid was filtered out to obtain 87.79 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt as a crude product.

6.84 g of maleic acid was added to 231 g of the crude product containing the crude product obtained above and those manufactured in a similar manner, dissolved in 5.5 L of 80% ethanol water, and then the precipitated solid was collected by filtering to obtain 203 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.

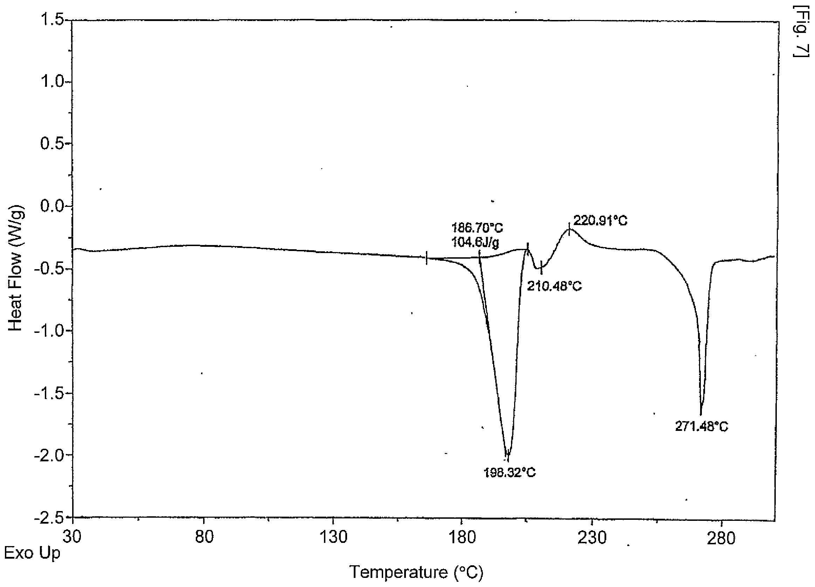

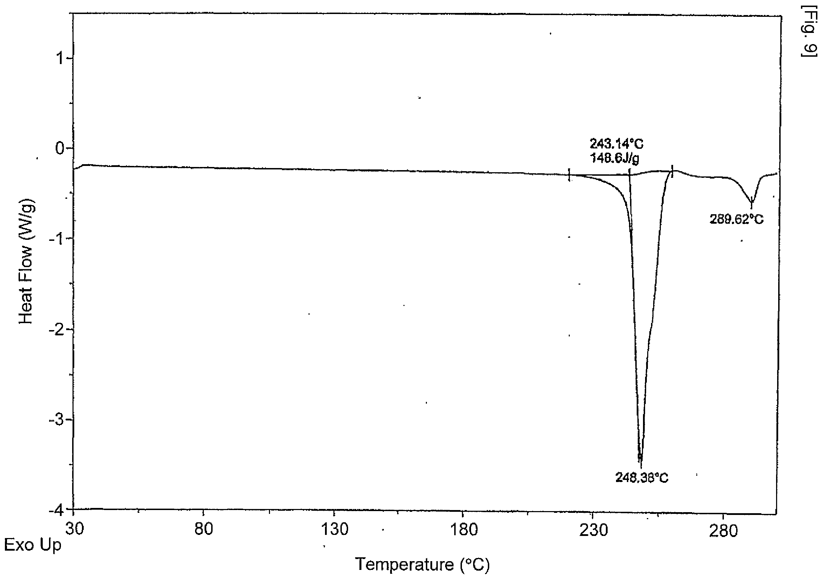

As shown in Preparation Example 1, when the preparation method of example 1 shown in patent literature 2 was scaled up, the compound X maleic acid salt crystals exhibiting the differential scanning calorimeter analysis chart presented in FIG. 9 were obtained, and were found to have different endothermic peaks when compared to the differential scanning calorimeter analysis chart of compound X maleic acid salt A type crystals presented in FIG. 7.

These results show that the compound X maleic acid salt A type crystals have a possibility that the crystal form will morph depending on the scale of production, and thus are clearly inappropriate as a drug substance for medicines which require constant quality and constant effects.

### Example 1: Manufacture of Compound X Maleic Acid Salt C Type Crystals (1)

1.52 L of ethanol, 0.38 L of water, and 15.7 g of maleic acid were added to 78.59 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid, and heated while stirring. After cooling to room temperature and further cooling with ice, the precipitated solid was collected by filtering to obtain 71.60 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt as a crude product.

296 mg of maleic acid was added to 10.0 g of the crude product obtained, dissolved in 60 mL of acetone, 60 mL of DMSO, and 30 mL of water, and then the precipitated solids were collected to obtain 8.41 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.

### Example 2: Manufacture of Compound X Maleic Acid Salt C Type Crystals (2)

A mixture containing 80.1 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid, 580mL of DMSO, 580 mL of acetone, 17.2 g of maleic acid, and 290 mL of water was stirred at 69 degree C. The insoluble matter was filtered out, washed with a mixture of 32 mL of DMSO, 32 mL of acetone, and 16 mL of water, and then the filtrate was cooled and the precipitate was collected by filtering. Washing was successively performed using 150 mL of water, 80 mL of acetone, 650 mL of water, and 80 mL of acetone, followed by drying, to obtain 70.66 g of1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.

### Example 3: Manufacture of Compound X Maleic Acid Salt C Type Crystals (3)

A mixture containing 20 kg of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid, 100 L of DMSO, 100 L of acetone, 4.29 kg of maleic acid, and 50 L of water is stirred at 65 degree C, and then the insoluble matter is filtered out and washed with a mixture of 8 L of DMSO, 8 L of acetone, and 4 L of water, and then the filtrate is cooled, the precipitate is collected by filtering, successively washed using 40 L of acetone, 100 L of water, and 40 L of acetone, and then dried to obtain approximately 20 kg of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.

### Test Example 2: Mouse Oral Administration Test

3 mg/kg of the test compound suspended in an aqueous solution of 0.5% methylcellulose was orally administered to male ICR mice. 2 hours after administration, blood was sampled from the inferior vena cava using 1/10 volume 3.8% sodium citrate solution as an anti-coagulating agent. The blood plasmas obtained by centrifugal separation at 12,000 RPM for 3 minutes were heated for 30 minutes at 56 degree C and then added to the human c-mpl-Ba/F3 cell proliferation test system described in test example 1, to obtain 10%, 0.3% or 0.1% blood plasma concentration. The cell proliferation activity was measured and the cell proliferation activity (%) was determined based on the maximum cell proliferation activity of each test compound being 100%. The results are shown in Table 1. Note, "NT" indicates that a measurement was not performed.

**Table 1 Human c-mpl-Ba/F3 cell proliferation activity of blood platelets after orally administering test compounds**

| Test Compound | Cell proliferation activity (%) | | |
|---|---|---|---|
| | 10% | 0.3% | 0.1% |
| Compound X hydrochloride | 34 | < 10 | NT |
| Compound X maleic acid salt A type crystal | >80 | 55 | NT |
| Compound X maleic acid salt B type crystal | NT | < 10 | NT |
| Compound X maleic acid salt C type crystal | NT | > 80 | 30 |

Compound X hydrochloride was manufactured in accordance with the method disclosed in patent literature 1. Furthermore, compound X maleic acid salt A type crystals were manufactured in accordance with the method disclosed in patent literature 2.

From the foregoing results, it was made clear that of the three types of crystals of compound X maleic acid salt, compound X maleic acid salt A type crystals and compound X maleic acid salt C type crystal had excellent oral absorption, but compound X maleic acid B type crystals had inferior oral absorption.

Therefore, the compound X maleic acid salt A type crystals had excellent oral absorption, but there is a possibility that the crystal form will morph depending on the scale of production, and therefore these crystals may be inappropriate as a drug substance for medicines which require constant quality and constant effects. Furthermore, the compound X maleic acid salt B type crystal can be stably supplied in mass production such as industrial production, but the oral absorption thereof was found to be inferior. On the other hand, compound X maleic acid salt C type crystals were the only crystal that had excellent oral absorbency and could be stably supplied in mass production such as industrial production.

### Industrial Applicability

Compound X maleic acid salt C type crystals exhibit excellent oral absorption, ensure constant quality even during mass production such as industrial production, and can be stably supplied. Furthermore, compound X maleic acid salt C type crystals can be used as an active ingredient for a pharmaceutical composition for treating thrombocytopenia.

## Claims

1. A crystal of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, wherein the endothermic peak top temperatures in differential scanning calorimeter analysis are between 229 and 232 degree C, and between 300 and 303 degree C.

2. A crystal of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, having peaks in powder x-ray crystal analysis using a Cu-K alpha beam at 8.5 degree, 9.5 degree, 12.1 degree, 20.4 degree, 22.7 degree, and 23.3 degree for a diffraction angle of 2-theta.

3. The crystal according to claim 2, having endothermic peak top temperatures in differential scanning calorimeter analysis of 229 to 232 degree C, and 300 to 303 degree C.

4. A pharmaceutical composition comprising the crystal according to any one of claims 1 to 3, and a pharmaceutically acceptable excipient.

5. The pharmaceutical composition according to claim 4, which is a pharmaceutical composition for preventing or treating thrombocytopenia.

6. Use of the crystal according to any one of claims 1 to 3, for producing a pharmaceutical composition for preventing or treating thrombocytopenia.

7. The crystal according to any one of claims 1 to 3, for preventing or treating thrombocytopenia.

## Patentansprüche

1. Kristall eines 1-(3-Chlor-5-{[4-(4-chlorthiophen-2-yl)-5-(4-cyclohexylpiperazin-1-yl)thiazol-2-yl]carbamoyl}-pyridin-2-yl)piperidin-4-carbonsäure-Maleinsäuresalzes, wobei die endothermen Peakspitzentemperaturen bei einer Differential-Scanning-Kalorimeteranalyse zwischen 229 und 232°C und zwischen 300 und 303°C liegen.

2. Kristall eines 1-(3-Chlor-5-{[4-(4-chlorthiophen-2-yl)-5-(4-cyclohexylpiperazin-1-yl)thiazol-2-yl]carbamoyl}-pyridin-2-yl)piperidin-4-carbonsäure-Maleinsäuresalzes, der Peaks in einer Pulver-Röntgen-Kristallanalyse unter Verwendung eines Cu-K-α-Strahls bei 8,5°, 9,5°, 12,1°, 20,4°, 22,7° und 23,3° für einen Beugungswinkel von 2θ aufweist.

3. Kristall gemäss Anspruch 2, der endotherme Peakspitzentemperaturen bei einer Differential-Scanning-Kalorimeteranalyse von 229 bis 232°C und 300 bis 303°C aufweist.

4. Pharmazeutische Zusammensetzung, die einen Kristall gemäss irgendeinem der Ansprüche 1 bis 3 und einen pharmazeutisch annehmbaren Hilfsstoff umfasst.

5. Pharmazeutische Zusammensetzung gemäss Anspruch 4, die eine pharmazeutische Zusammensetzung zur Prävention oder Behandlung von Thrombocytopenie ist.

6. Verwendung eines Kristalls gemäss irgendeinem der Ansprüche 1 bis 3 zur Herstellung einer pharmazeutischen Zusammensetzung zur Prävention oder Behandlung von Thrombocytopenie.

7. Kristall gemäss irgendeinem der Ansprüche 1 bis 3 zur Prävention oder Behandlung von Thrombocytopenie

## Revendications

1. Cristal d'un sel d'acide maléique d'acide 1-(3-chloro-5-{[4-(4-chlorothiophèn-2-yl)-5-(4-cyclohexyl pipérazin-1-yl)thiazol-2-yl]carbamoyl}pyridin-2-yl)pipéridine-4-carboxylique, dans lequel les températures supérieures de pic endothermiques dans une analyse au calorimètre à balayage différentiel sont situées entre 229 et 232 degrés C, et entre 300 et 303 degrés C.

2. Cristal d'un sel d'acide maléique d'acide 1-(3-chloro-5-{[4-(4-chlorothiophèn-2-yl)-5-(4-cyclohexyl pipérazin-1-yl)thiazol-2-yl]carbamoyl}pyridin-2-yl)pipéridine-4-carboxylique, présentant des pics dans une analyse de cristal par diffraction de rayons X sur poudre en utilisant un rayonnement Cu K-alpha à 8,5 degrés, 9,5 degrés, 12,1 degrés, 20,4 degrés, 22,7 degrés, et 23,3 degrés pour un angle de diffraction de 2 thêta.

3. Cristal selon la revendication 2, présentant des températures supérieures de pic endothermique dans une analyse au calorimètre à balayage différentiel de 229 à 232 degrés C, et de 300 à 303 degrés C.

4. Composition pharmaceutique comprenant le cristal selon l'une quelconque des revendications 1 à 3, et un excipient acceptable du point de vue pharmaceutique.

5. Composition pharmaceutique selon la revendication 4, qui est une composition pharmaceutique destinée à être utilisée dans la prévention ou le traitement de la thrombocytopénie.

6. Utilisation du cristal selon l'une quelconque des revendications 1 à 3, pour l'obtention d'une composition pharmaceutique destinée à une utilisation de prévention ou de traitement de la thrombocytopénie.

7. Cristal selon l'une quelconque des revendications 1 à 3, destiné à être utilisé dans la prévention ou le traitement de la thrombocytopénie.
